# EUROPEAN PATENT SPECIFICATION

(11) **EP 0 373 827 B1**
(45) Date of publication and mention of the grant of the patent: **27.10.1993**
(21) Application number: 89312736.5
(22) Date of filing: 07.12.1989
(51) Int. Cl.: A61K 31/505

(54) **Derivatives of 5-hydroxy and 5-methoxy 2-amino-pyrimidines as inhibitors of interleukin-1 production**
Derivate von 5-Hydroxy- und 5-Methoxy-2-amino-pyrimidin als Inhibitoren der Interleukin-1-Produktion
Dérivés de 5-hydroxy et 5-méthoxy-2-amino-pyrimidines comme inhibiteurs de la production d'interleukine-1

(30) Priority: 14.12.1988 US 284505
(43) Date of publication of application: 20.06.1990
(73) Proprietor: PFIZER INC., New York, N.Y. 10017 (US)
(72) Inventor: Walker, Frederick Judson, Preston Connecticut (US)
(74) Representative: Moore, James William, Dr.

(56) References cited:
- EP-A- 0 186 405
- GB-A- 1 189 188
- US-A- 4 711 888
- J. of Clinical Immunology, vol. 5, no. 5, 1985, pp. 287-293, Plenum Publishing Corp.; C.A. Dinarello
- The J. of Immunology, vol. 137, no. 11, 1st Dec. 1986, pp. 3469-3474, The Am. Assoc. of Immunologists; R.D.R. Camp et al.
- J. Heterocycl. Chem., vol. 15, no. 7, 1978, pp. 1097-1099; A. Kreutzberger et al.

## Description

This invention relates to the use of certain derivatives of 5-hydroxy and 5-methoxy 2-amino-pyrimidines and the pharmaceutically-acceptable salts thereof to inhibit interleukin-1 production in a mammal. This invention also relates to the use of such compounds for treating interleukin-1 mediated disorders and dysfunctions such as bone and connective tissue metabolism disorders and immune dysfunction in a mammal. The methods of this invention comprise administering an effective amount of the compounds and salts of this invention to such a mammal.

Certain 5-hydroxy and 5-alkoxy pyrimidines of the formula 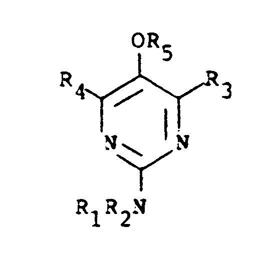
and the pharmaceutically-acceptable salts thereof wherein, inter alia, R₁ is H; R₂ is H, (C₁-C₁₅) alkyl or (C₇-C₂₀)phenylalkyl which may be substituted in the phenyl by one or two of fluoro or chloro; R₃ is (C₁-C₆)alkyl; R₄ is H or (C₁-C₆)alkyl; and R₅ is H or (C₁-C₆)alkyl are disclosed and claimed in U.S. 4,711,888. That patent discloses that those compounds are inhibitors of leukotriene synthesis and are useful for the treatment of pulmonary, inflammatory, allergic and cardiovascular diseases as well as being cytoprotective and, therefore, useful in the treatment of peptic ulcers. Specific utilities disclosed for those compounds include treatment of asthma, bronchitis, pulmonary diseases, such as pulmonary hypertension and hypoxia, peptic ulcers, psoriasis, arthritis, inflammatory bowel disease or cardiovascular spasm, such as acute myocardial infarctions.

Certain 2-amino-5-hydroxy-4-methyl pyrimidine derivatives of the formula 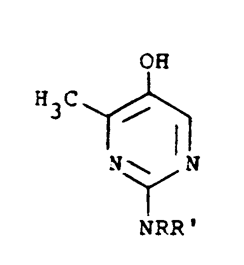
and the pharmaceutically-acceptable acid addition salts thereof wherein, inter alia, R is H or (C₁-C₁₅)alkyl and R' is (C₁-C₁₅)alkyl are disclosed and claimed in U.S. 4,554,276. That patent discloses that those compounds are inhibitors of leukotriene synthesis and are useful in the treatment of pulmonary, inflammatory and cardiovascular diseases, cancer and psoriasis. Further, those compounds are disclosed as having cytoprotective activity and therefore are also useful in the treatment of peptic ulcers. A method of treating gastrointestinal disorders with compounds disclosed in U.S. 4,554,276 discussed above is disclosed and claimed in U.S. 4,673,677.

Interleukin-1 (IL-1) has been reported to stimulate bone resorption both in vitro and in vivo. Hayward, M. and Fiedler-Nagy, Ch., Agents and Actions, 22, 251-254 (1987). It is also reported therein that IL-1, inter alia, induces the production of prostaglandin E₂ (PGE₂). PGE₂ is a stimulator of bone resorption and has been implicated in bone loss. Hayward, M. A. and Caggiano, T. J., Annual Reports in Medicinal Chemistry, 22, Sect. IV, Chapter 17, 172-177 (1987). Osteoporosis is defined as a debilitory loss of bone mineral which results in higher fracture rates. See Hayward, M. A. and Caggiano, T. J., supra and references cited therein.

Interleukin-1 has been reported to be involved in the pathogenesis of many diseases. See Dinarello, C. A., J. Clin. Immunol., 5, 287-297 (1985), the teachings of which are incorporated herein by reference. Further still, elevated levels of IL-1 like material have been found to be associated with psoriasis. Camp, R. D., et al., J. Immunol., 137, 3469-3474 (1986).

The non-steroidal anti-inflammatory agent etodolac, 1,8-diethyl-1,3,4,9-tetrahydropyrano[3,4-b]indole-1-acetic acid, has been disclosed in U.S. 4,677,132 to lower PGE₂ and reduce bone resorption. Etodolac has the formula

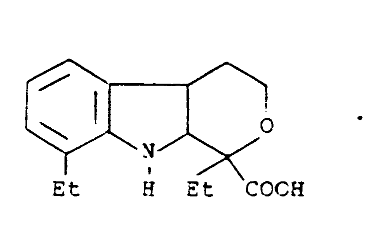

It has been reported that therapeutic levels of nonsteroidal anti-inflammatory agents such as indomethacin and ibuprofen do not reduce IL-1 production. Similarly, cyclosporine A had no such effect. Corticosteroids, however, are effective in reducing IL-1 production. Dinarello, C. A., supra. Certain lipoxygenase inhibitors such as 5,8,11,14-eicosatetraynoic acid (ETYA) and 3-amino-1,3-trifluoromethylphenyl-2-pyrazoline (BW755C) have been reported to decrease in vitro production of leukocytic pyrogen (putative IL-1) from human monocytes. Dinarello, C. A., et al., Int. J. Immunopharmac., 6, 43-50 (1984).

However, until the invention herein, there was no report of use or intent to use the compounds or salts of this invention to inhibit IL-1 production and to treat IL-1 mediated disorders and dysfunctions such as certain bone and connective tissue metabolism disorders and certain immune dysfunctions with such compounds nor any appreciation of their role in such treatments.

It has been found that certain 5-hydroxy and 5-methoxy pyrimidines of the formula 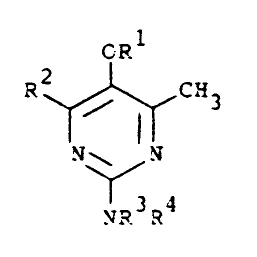
and the pharmaceutically-acceptable salts thereof wherein R¹ is H or CH₃; R² is CH₃; R³ is (C₃-C₁₅) straight chain alkyl or (C₇-C₂₀)phenylalkyl which may be substituted in the phenyl by one or two fluoro or chloro substituents; and R⁴ is H or CH₃ inhibit the production of IL-1 and thus are useful in treating IL-1 mediated disorders and dysfunctions such as certain disorders of bone and connective tissue metabolism and dysfunctions of the autoimmune system in mammals. Such bone metabolism disorders include, but are not limited to osteoporosis. By way of example and not of limitation, such connective tissue metabolism disorders include periodontal disease and tissue scarring. Further, examples of IL-1 mediated immune dysfunctions include, but are not limited to, allergy and psoriasis.

The methods of using the compounds and their pharmaceutically-acceptable base salts comprise administering to a mammal an effective amount of such compounds. Administration can comprise any known method for therapeutically providing a compound to a mammal such as by oral or parenteral administration as defined hereinbelow.

The compounds which are used according to this invention which are of the formula 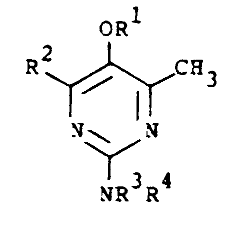
and the pharmaceutically-acceptable salts thereof wherein R¹ is H or CH₃; R² is CH₃; R³ is (C₃-C₁₅) straight chain alkyl or (C₇-C₂₀)phenylalkyl which may be substituted in the phenyl by one or two fluoro or chloro substituents; and R⁴ is H or CH₃ and the preparation thereof are disclosed in U.S. 4,554,276 and U.S. 4,711,888.

Of the uses described above, preferred therein are those where the compound employed is of the formula above wherein R¹ is H; R² is CH₃; R³ is (CH₂)₈CH₃ or (CH₂)₆C₆H₅ and R⁴ is H; those wherein in said compound R¹ is H, R² is H; R³ is (CH₂)₆C₆H₅; and R⁴ is H; and those wherein in said compound R¹ is CH₃; R² is CH₃; R³ is (CH₂)₆C₆H₅; and R⁴ is CH₃. Particularly preferred are methods wherein in said compound R¹ is H; R² is CH₃; R³ is (CH₂)₆C₆H₅; and R⁴ is H.

As disclosed in U.S. 4,554,276 and U.S. 4,711,888 the compounds of this invention hereinabove described will form pharmaceutically-acceptable salts. All such pharmaceutically-acceptable salts are within the scope of this invention and can be formed as taught by those patents. Such suitable salts, within the scope of this invention, include both the organic and inorganic types. Preferred acid addition salts are those of acetic, ascorbic, lactic, sulfonic, hydrobromic, hydroiodic and hydrochloric. Particularly preferred are the salts of sodium wherein R¹ is H; hydrochloride; phosphate; and tartrate.

Interleukin-1 is known by those skilled in the art to exist in at least two forms which are referred to as the α and β forms. Dinarello, C. A., FASEB J., 2, 108-115 (1988). As used throughout this specification and the appendant claims, the term interleukin-1 (IL-1) refers to all such forms of IL-1 including IL-1α, IL-1β and Il-1α and IL-1β collectively.

The compounds and their salts can be administered to said mammal either alone or, preferably, in combination with pharmaceutically-acceptable carriers or diluents in a pharmaceutical composition, according to standard pharmaceutical practice. Such administration can be oral or parenteral. Parenteral administration as used herein includes, but is not limited to, intravenous, intramuscular, intraperitoneal, subcutaneous, transdermal, transmucosal and topical including, but not limited to, oral lavage administration. However, it is generally preferred to administer such compounds and their salts topically.

In general, these compounds and their salts are most desirably administered in doses ranging from about 0.01 mg up to about 100 mg/kg body weight of the subject to be treated per day, although variations will still necessarily occur depending upon the subject being treated. The appropriate dose for inhibiting IL-1 production in a mammal and for treatment of IL-1 mediated bone metabolism disorder, IL-1 mediated connective tissue metabolism disorder or IL-1 mediated immune dysfunction with the compounds and their salts of this invention will be readily determined by those skilled in the art of prescribing and/or administering such compounds. Nevertheless, it is still to be appreciated that other variations may also occur in this respect, depending upon the species of mammal being treated and its individual response to said medicament, as well as on the particular type of pharmaceutical formulation chosen and the time period and interval at which such administration is carried out. In some instances, dosage levels below the lower limit of the aforesaid range may be more than adequate, while in other cases still larger doses may be employed without causing any harmful or deleterious side effects to occur, provided that such higher dose levels are first divided into several smaller doses that are to be administered throughout the day.

For purposes of oral administration, tablets containing excipients such as sodium citrate, calcium carbonate and dicalcium phosphate may be employed along with various disintegrants such as starch and preferably potato or tapioca starch, alginic acid and certain complex silicates, together with binding agents such as polyvinylpyrrolidone, sucrose, gelatin and acacia. Additionally, lubricating agents such as, but not limited to, magnesium stearate, sodium lauryl sulfate and talc are often very useful for tableting purposes. Solid compositions of a similar type may also be employed as fillers in soft elastic and hard-filled gelatin capsules; preferred materials in this connection also include, by way of example and not of limitation, lactose or milk sugar as well as high molecular weight polyethylene glycols. When aqueous suspensions and/or elixirs are desired for oral administration, the essential active ingredient may be combined with various sweetening or flavoring agents, coloring matter or dyes and, if so desired, emulsifying and/or suspending agents, together with diluents such as water, ethanol, propylene glycol, glycerin and various like combinations thereof.

Although the preferred mode of administration of the compounds of this invention and their pharmaceutically-acceptable salts is oral, they may also be administered parenterally.

For purposes of parenteral administration, solutions of these particular compounds in sesame or peanut oil or in aqueous propylene glycol may be employed, as well as sterile aqueous solutions of the corresponding water soluble salts thereof. Such aqueous solutions should be suitably buffered, if necessary, and the liquid diluent rendered isotonic with sufficient saline or glucose. These particular aqueous solutions are especially suitable for intravenous, intramuscular and subcutaneous injection purposes. In this connection, the sterile aqueous media employed are readily obtained by standard techniques well known to those skilled in the art. For instance, distilled water is ordinarily used as the liquid diluent and the final preparation is passed through a suitable bacterial filter such as a sintered glass filter or a diatomaceous-earth or unglazed porcelain filter. Preferred filters of this type include the Berkefeld, the Chamberland and the Asbestos Disk-Metal Seitz filter, wherein the fluid is sucked into a sterile container with the aid of a suction pump. Of course, the necessary steps should be taken throughout the preparation of these injectable solutions to insure that the final products are obtained in a sterile condition. For purposes of transdermal administration, the dosage form of the particular compound may include, by way of example, solutions, lotions, ointments, creams, gels, suppositories, rate-limiting sustained release formulations and devices therefor. Such dosage forms comprise the particular compound and may include ethanol, water, penetration enhancer and inert carriers such as gel-producing materials, mineral oil, emulsifying agents, benzyl alcohol and the like. Specific transdermal flux enhancing compositions are disclosed in European Patent Application, publication number 271983, published June 22, 1988. For purposes of topical administration, the dosage form of the particular compound may include, by way of example and not of limitation, solutions, lotions, ointments, creams and gels.

The ability of the compounds of this invention to inhibit interleukin-1 biosynthesis is demonstrated by the assay procedures described below.

THP-1 cells [ATCC TIB 202; Int. J. Cancer 26:171 (1980); Cancer Res. 42:15230 (1982); J. Immunol. 131:1882 (1983); Biochemistry 25:2424 (1986)] are maintained in RPMI medium (Hazelton Research Products, Inc., Lenexa, Kansas) with 10% fetal bovine serum, 2mM glutamine, 100 µ/ml penicillin and 100 µg/ml streptomycin at 37° C. under an atmosphere of 95% oxygen and 5% carbon dioxide. The compound under study is dissolved initially in DMSO and diluted to a final concentration of 1% DMSO in RPMI. A known concentration of the compound prepared as described above is added in a volume of 0.25 ml to a well of a 24-well multicluster tissue culture plate. The THP-1 cells maintained as described above are harvested by centrifugation in 50 ml conical tubes at 500xg and room temperature. The resulting cell pellet is resuspended and washed twice with serum free RPMI containing glutamine and antibiotics as described above. The cells are suspended to a final density of 2x10⁶ cells/ml. Then, fetal bovine serum is added to the cell suspension to a final concentration of 0.65 weight percent. The THP-1 cells are then stimulated with lipopolysaccharide (LPS) from Salmonella minnesota and silica by adding LPS and silica from stock solutions of each, prepared at 1 mg/ml in RPMI, to a final concentration of 13 µg/ml for each. A volume of 0.75 ml of the stimulated cell suspension is added to each well of the multicluster tissue culture plate containing a solution of the compound under study or RPMI medium as a control. The plates are mixed and incubated at 37° C. for 24 hours under an atmosphere of 95% oxygen and 5% carbon dioxide. After incubation, the medium from each well is centrifuged separately at 1500xg and the supernatants recovered. The supernatants are then assayed for IL-1β immediately or, if not, stored at -70° C. until assayed. The IL-1β concentration in the medium is determined by a sandwich ELISA system obtained from Cistron Biotechnology (10 Bloomfield Avenue, Line Brook, New Jersey 07058) according to the procedure specified by the manufacturer. The resulting color reaction of the conjugated horseradish peroxidase enzyme conversion of o-phenylenediame to o-quinone is read at 490 nm on an ELISA plate reader and is proportional to the amount of IL-1β present in the sample as determined from a standard curve using an IL-1β standard and is expressed in pg/ml.

## Claims

1. The use of a compound of the formula 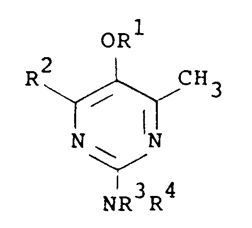 or a pharmaceutically-acceptable salt thereof, wherein R¹ is H or CH₃; R² is CH₃; R³ is (C₃-C₁₅) straight chain alkyl or (C₇-C₂₀)phenylalkyl which may be substituted in the phenyl by one or two fluoro or chloro substituents; and R⁴ is H or CH₃, for the preparation of a pharmaceutical composition for inhibiting interleukin-1 production in a mammal, in need thereof.

2. The use of a compound according to claim 1 wherein the pharmaceutical composition is for the treatment of interleukin-1 mediated bone metabolism disorders in a mammal.

3. The use of a compound or a pharmaceutically-acceptable salt thereof according to claim 2 wherein the bone metabolism disorder is osteoporosis.

4. The use of a compound according to claim 1 wherein the pharmaceutical composition is for the treatment of interleukin-1 mediated connective tissue metabolism disorders in a mammal.

5. The use of a compound or a pharmaceutically-acceptable salt thereof according to claim 4 wherein the connective tissue metabolism disorder is periodontal disease or tissue scarring.

6. The use of a compound according to claim 1 wherein the pharmaceutical composition is for the treatment of interleukin-1 mediated immune dysfunction in a mammal.

7. The use of a compound or a pharmaceutically-acceptable salt thereof according to any one of claims 1-6 wherein R¹ is H; R² is CH₃; R³ is (CH₂)₆C₆H₅; and R⁴ is H.

8. The use of a compound or a pharmaceutically-acceptable salt thereof according to any one of claims 1-6 wherein R¹ is H; R² is CH₃; R³ is (CH₂)₈CH₃; and R⁴ is H.

9. The use of a compound or a pharmaceutically-acceptable salt thereof according to any one of claims 1-6 wherein R¹ is CH₃; R² is CH₃; R³ is (CH₂)₆C₆H₅; and R⁴ is CH₃.

10. The use of a compound or a pharmaceutically-acceptable salt thereof according to any one of claims 1-9 wherein the pharmaceutical composition is suitable for oral or parenteral administration.

## Patentansprüche

1. Verwendung einer Verbindung der Formel 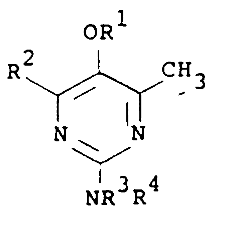 oder eines pharmazeutisch verträglichen Salzes davon, worin R¹ ein Wasserstoffatom oder CH₃ bedeutet; R² CH₃ bedeutet; R³ eine geradkettige (C₃-C₁₅)-Alkyl- oder (C₇-C₂₀)-Phenylalkylgruppe, gegebenenfalls substituiert in der Phenylgruppe mit ein oder zwei Fluor- oder Chlorsubstituenten, bedeutet und R⁴ ein Wasserstoffatom oder CH₃ bedeutet, zur Herstellung eines Arzneimittels zur Inhibierung der Interleukin-1-Erzeugung bei betroffenen Säugern.

2. Verwendung einer Verbindung nach Anspruch 1, wobei das Arzneimittel zur Behandlung von Interleukin-1-vermittelten Knochenmetabolismuserkrankungen bei Säugern vorgesehen ist.

3. Verwendung einer Verbindung oder eines pharmazeutisch verträglichen Salzes davon nach Anspruch 2, wobei die Knochenmetabolismuserkrankung Osteoporose ist.

4. Verwendung einer Verbindung nach Anspruch 1, wobei das Arzneimittel zur Behandlung Interleukin-1-vermittelter Metabolismuserkrankungen des Bindegewebes bei Säugern vorgesehen ist.

5. Verwendung einer Verbindung oder eines pharmazeutisch verträglichen Salzes davon nach Anspruch 4, wobei die Metabolismuserkrankung des Bindegewebes eine periodontale Erkrankung oder Gewebsvernarbung ist.

6. Verwendung einer Verbindung nach Anspruch 1, wobei das Arzneimittel zur Behandlung von Interleukin-1-vermittelter Immundysfunktion bei Säugern vorgesehen ist.

7. Verwendung einer Verbindung oder eines pharmazeutisch verträglichen Salzes davon nach einem der Ansprüche 1 bis 6, wobei R¹ ein Wasserstoffatom bedeutet; R² CH₃ bedeutet; R³ (CH₂)₆C₆H₅ darstellt und R⁴ ein Wasserstoffatom bedeutet.

8. Verwendung einer Verbindung oder eines pharmazeutisch verträglichen Salzes davon nach einem der Ansprüche 1 bis 6, wobei R¹ ein Wasserstoffatom bedeutet; R² CH₃ bedeutet; R³ (CH₂)₈CH₃ darstellt und R⁴ ein Wasserstoffatom bedeutet.

9. Verwendung einer Verbindung oder eines pharmazeutisch verträglichen Salzes davon nach einem der Ansprüche 1 bis 6, wobei R¹ CH₃ bedeutet; R² CH₃ bedeutet; R³ (CH₂)₆C₆H₅ darstellt und R⁴ CH₃ bedeutet.

10. Verwendung einer Verbindung oder eines pharmazeutisch verträglichen Salzes davon nach einem der Ansprüche 1 bis 9, wobei das Arzneimittel zur oralen oder parenteralen Verabreichung geeignet ist.

## Revendications

1. Utilisation d'un composé de formule 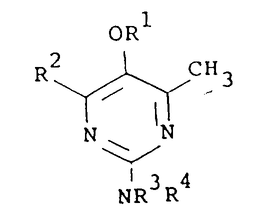 ou d'un sel pharmaceutiquement acceptable de ce composé, formule dans laquelle R¹ représente H ou CH₃ ; R² est un groupe CH₃ ; R³ est un groupe alkyle en C₃ à C₁₅ à chaîne droite ou un groupe phénylalkyle en C₇ à C₂₀ qui peut être substitué dans la partie phényle par un ou deux substituants fluoro ou chloro ; et R⁴ représente H ou CH₃, pour la préparation d'une composition pharmaceutique destinée à inhiber la production d'interleukine-1 chez un mammifère qui le nécessite.

2. Utilisation d'un composé suivant la revendication 1, dans laquelle la composition pharmaceutique est destinée au traitement de troubles du métabolisme des os sous la médiation de l'interleukine-1 chez un mammifère.

3. Utilisation d'un composé ou d'un sel acceptable du point de vue pharmaceutique d'un composé suivant la revendication 2, dans laquelle le trouble du métabolisme des os est l'ostéoporose.

4. Utilisation d'un composé suivant la revendication 1, dans laquelle la composition pharmaceutique est destinée au traitement de troubles du métabolisme du tissu conjonctif sous la médiation de l'interleukine-1 chez un mammifère.

5. Utilisation d'un composé ou d'un sel pharmaceutiquement acceptable d'un composé suivant la revendication 4, dans laquelle le trouble du métabolisme du tissu conjonctif est la parodontolyse ou la cicatrisation du tissu.

6. Utilisation d'un composé suivant la revendication 1, dans laquelle la composition pharmaceutique est destinée au traitement d'un disfonctionnement immunitaire sous la médiation de l'interleukine-1 chez un mammifère.

7. Utilisation d'un composé ou d'un sel acceptable du point de vue pharmaceutique d'un composé suivant l'une quelconque des revendications 1 à 6, dans laquelle R¹ représente H ; R² représente CH₃, R³ est un groupe (CH₂)₆C₆H₅ et R⁴ représente H.

8. Utilisation d'un composé ou d'un sel pharmaceutiquement acceptable d'un composé suivant l'une quelconque des revendications 1 à 6, dans laquelle R¹ représente H ; R⁶ représente CH₃ ; R³ est un groupe (CH₂)₈CH₃ . et R⁴ représente H.

9. Utilisation d'un composé ou d'un sel pharmaceutiquement acceptable d'un composé suivant l'une quelconque des revendications 1 à 6, dans laquelle R¹ représente CH₃, R² représente CH₃ ; R³ représente (CH₂)₆C₆H₅ ; et R⁴ représente CH₃.

10. Utilisation d'un composé ou d'un sel pharmaceutiquement acceptable d'un composé suivant l'une quelconque des revendications 1 à 9 dans laquelle la composition pharmaceutique convient pour l'administration orale ou parentérale.
